Europäisches Patentamt

European Patent Office

Office européen des brevets

(11) Publication number: **0 371 658**
**A2**

# EUROPEAN PATENT APPLICATION

(21) Application number: 89311860.4

(22) Date of filing: 16.11.89

(51) Int. Cl.⁵: **C12N 9/08,** //**(C12N9/08,**
**C12R1:645)**

(30) Priority: 16.11.88 US 272024

(43) Date of publication of application:
06.06.90 Bulletin 90/23

(84) Designated Contracting States:
**DE FR GB**

(71) Applicant: **EASTMAN KODAK COMPANY**
**343 State Street**
**Rochester, New York 14650(US)**

(72) Inventor: **Ciccarelli, Richard Benjamin**
**EASTMAN KODAK COMPANY**
**Patent Department 343 State Street**
**Rochester New York 14650(US)**

(74) Representative: **Nunney, Ronald Frederick**
**Adolphe et al**
**Kodak Limited Patent Department Headstone**
**Drive**
**Harrow Middlesex HA1 4TY(GB)**

(54) **Purification of cytochrome C peroxidase from yeast.**

(57) A method of purifying cytochrome c peroxidase from yeast is disclosed. The method comprises the steps of:

a) extracting a yeast lysate to obtain a crude protein extract comprising cytochrome c peroxidase;

b) differentially precipitating the crude protein extract to obtain a precipitate comprising CCP;

c) Carrying out anion exchange chromatography on precipitate obtained in b) to obtain partially pure CCP; and

d) carrying out phosphate affinity chromatography, with a hydroxyapatite column, on the partially pure CCP to obtain pure CCP.

## PURIFICATION OF CYTOCHROME C PEROXIDASE FROM YEAST

The present invention relates to a method of purifying cytochrome c peroxidase extracted from yeast.

Peroxidases are enzymes which are used commercially as reagents in the quantitative analysis of a number of chemicals such as hydrogen peroxide and glucose. Peroxidases are used as labels in enzyme immunoassays.

Cytochrome c peroxidase is an example of a peroxidase enzyme. It is used, inter alia, in methods and elements for detecting oxidase positive organisms. Cytochrome c peroxidase (CCP, Cytochrome c:$H_2O_2$ oxidoreductase, E.C.1.11.1.5) is an enzyme which exists in the mitochondria in baker's yeast (Sacharomyces cerevisiae). It catalyzes the peroxide-mediated oxidation of ferrocytochrome c:

$$2\ \text{CYTc}(Fe^{2+}) + H_2O_2 + 2H^+ \xrightarrow{\text{CCP}} 2\ \text{CYTc-}(Fe^{3+}) + 2\ H_2O$$

Yeast CCP is a single chain protein of 294 amino acids (MW = 34,000 d) which contains one mole of non-covalently attached heme (iron protoporphyrin IX) per mole of protein. CCP is an acidic protein (pH = 5.3) with known primary and tertiary structural information.

It is known that cytochrome c peroxidase can be extracted from baker's yeast. The most efficient prior art method of which the inventor is aware is disclosed by Nelson et al in a paper entitled "Preparation of Cytochrome c Peroxidase From Baker's Yeast", Analytical Biochemistry, 83, 622-631 (1977). The method comprises a) partially removing water from pressed yeast cakes; b) autolysis of the yeast; c) extraction into a buffer; d) binding the enzyme onto a diethyl amino ethyl (DEAE) cellulose anion exchange resin (DE-52) at moderate high ionic strength; e) eluting an impure CCP fraction; f) concentrating the fraction and g) subjecting the concentrate to gel filtration in a pH 5 sodium acetate buffer. The enzyme is then crystallized from an impure fraction by dialysis. The Nelson procedure suffers from several problems. First, yeast proteases degrade cytochrome c peroxidase (CCP) thereby decreasing potential CCP yield. Second, the method involves passing a solution containing the enzyme through a DE-52 microgranular anion exchange resin packed on a 4 L centered glass Buchner funnel. This anion exchange resin removes the heme active site from 10-20% of the enzyme molecules, which are thereby denatured. Third, the Nelson preparation takes 7 to 10 days to complete and involves three column chromatography steps in addition to several intermediate steps. Finally, this method requires a time-consuming dialysis and crystallization step which results in impure CCP crystals.

The present invention provides a method of purifying cytochrome c peroxidase from baker's yeast requiring less than 7 to 10 days with increased yields from dried yeast. The method is characterized by the steps of:

a) extracting a yeast lysate to obtain a crude protein extract comprising cytochrome c peroxidase;

b) differentially precipitating the crude protein extract to obtain a precipitate comprising CCP with fewer other proteins;

c) carrying out anion exchange chromatography on precipitate obtained in b) to obtain partially pure CCP; and

d) carrying out phosphate affinity chromatography, with a hydroxylapatite column, on the partially pure CCP to obtain pure CCP.

An especially useful embodiment of the foregoing method of this invention comprises the steps of:

a) lysing the yeast;

b) extracting the resulting lysate with a buffer having a pH of at least 5;

c) treating the extract with increasing concentrations of ammonium sulfate solutions up to at least 80 % W/V to obtain differential salt precipitation of proteins in extract;

d) dissolving protein containing precipitate obtained with at least 80% W/V ammonium sulfate solution in a buffer having i) a pH of at least 5 and ii) protease inhibitors dissolved therein;

e) lowering the salt concentration in the solution of d);

f) binding the proteins from the solution onto an anion exchange column;

g) washing the column with a buffer having a pH of at least 5 until the washings are free of proteins;

h) eluting the proteins from the column with a buffer having i) a pH of at least 5 and ii) a salt gradient concentration range of from 0.2 to at least 0.5 M;

i) lowering salt concentration in the eluates from h) having CCP activity;

j) binding protein from the eluates of i) onto a hydroxyapatite column; and

k) eluting pure CCP from the hydroxylapatite column with a linear phosphate gradient having a pH of 6 to 8 and a concentration ranging up to at least 0.4 M.

The method of this invention requires 2 to 3 days to complete. It extracts pure cytochrome c peroxidase in yields which are 50% greater than achieved in the Nelson et al method.

The method of this invention has several advantages over prior art methods in that a) the method utilizes the technique of differential salt fractionation as a preliminary purification step, which allows the final purification to be accomplished in just two additional steps; b) the method utilizes an anion exchange resin that gives superior resolution and superior yield of heme-containing enzyme at high flow rate, versus the conventional DE52 (Whatman) anion exchange resin that removes heme and denatures CCP; c) the method utilizes a linear salt gradient to elute CCP from b), which yields an 80% pure fraction after only one column step from crude lysate; d) the procedure utilizes a hydroxylapatite resin, for the final purification step, which gives a homogeneous CCP fraction (100%) in just two column steps from the crude lysate and e) the new procedure yields 20-40 mg/kg of highly purified and fully active CCP in just 2 to 3 days, which is much faster by several days than the conventional literature preps.

In particular useful embodiments of the method, dried yeast is autolysed in the presence of protease inhibitors that inhibit protease degradation of CCP.

The method extracts CCP from commercial strains of baker's yeast (Sacharomyces cerevisiae) available from a number of different commercial sources as a dried, granular solid or powder. However the method can be carried out on yeast having absorbed water.

## Step 1 - Extracting Yeast Lysate

CCP is located in yeast mitochondria. Preferably, whole yeast cells and mitochondria are lysed by the following procedure. The yeast, preferably dried, is mixed with cold (4°C) phosphate buffer at pH 6. The mixture is 1 Kg of yeast per 0.5 L buffer. Phosphate buffer is a mixture of mono-basic and di-basic sodium or potassium phosphate. It is a standard biological buffer. However, any buffer having the required pH can be used in carrying out this step. To prepare the buffer the mono- and dibasic sodium phosphate salts are titrated, one against the other, until the desired pH of 5 or greater is obtained. In addition, the phosphate buffer includes ethylenedinitrilo tetraacetic acid (EDTA) metal chelator and phenylmethylsulfonyl chloride (PMSF), which are both used to inhibit protease action on CCP. Next 0.5 L of ice cold ethyl acetate is added. The yeast and buffer are mixed to a uniform paste.

Under these conditions the yeast cells will autolyse. They will break apart without use of techniques for mechanical disruption of the cell walls, although such techniques could be used. The mixture is allowed to sit at 4°C for 6 hours. The yeast cells are completely lysed after this time.

To extract the crude protein containing the CCP from the lysate, another 1 L of the buffer is added to the lysate. Extraction can be carried out at a pH of at least 5. To extract all of the enzyme from the inside of the cells 1.5 L of the buffer is not used initially because autolysis would not occur. The proportions for the autolysing step should be about 1:1 buffer to ethyl acetate to treat 1 kg of dried yeast. The extraction proceeds by just adding 1 L of the cold buffer with stirring. In this extraction step all of the aqueous soluble material (including soluble proteins) is extracted from the cells into the buffer. All of the debris from yeast will not be soluble. After the extraction proceeds for 4 hours, the extract is centrifuged at 13,000 x g which removes all insoluble debris. Of course other separation techniques could be used to remove the debris. The supernatant will contain all of the CCP activity.

Fatty material will be floating on top of the supernatant. This fatty material contains lipids from the cell. The lipids are removed by filtering through, for example cheesecloth. To increase yields of protein, the debris fraction is sometimes resuspended in buffer, centrifuged a second time and filtered through cheesecloth. All of the supernatants are combined.

## Differential Salt Precipitation Step

Purification begins at this point in the method. Preferably, first, a technique known as ammonium sulfate salt precipitation or differential salt precipitation is applied to the combined supernatants. Any salt capable of forming at least an 80% W/V aqueous solution can be used. Different proteins are more or less soluble in different salt concentrations. There are a number of different proteins in the supernatant. By increasing the ammonium sulfate concentration, different proteins will precipitate out of the supernate solution at different salt concentrations. Experiments have shown that CCP will precipitate in a 80% (W/V), or above, of the salt ammonium sulfate aqueous solution at 4°C.

In carrying out this step first, ammonium sulfate is slowly added, while stirring, to the combined supernatant up to 50% W/V. Then the supernatant is set for an hour or two at a temperature of 4°C. All of the proteins that will come out at the 50% concentration or below will precipitate. CCP will remain in solution. The precipitate is removed.

Next the concentration of the salt in the supernatant is increased up to 80% W/V or above. Addition of salt in the supernatant at this salt concentration causes the ethyl acetate to become immiscible in the aqueous buffer. Hence two separate solvent layers are formed. Generally the two layers are physically separated and centrifuged separately. The pellets obtained from both centrifugations contain CCP. They are combined.

The pellets also contain significant amounts of contaminating salt which must be removed because the salt interferes in the anion exchange chromatography steps. Any technique may be used to lower the salt concentration. For example, the salt concentration could be lowered by adding sufficient water or buffer to the pellets to dilute the salt concentration to a level such that the salt cannot effectively compete with CCP for position on the anion exchange column.,

Alternatively, the pellets are dissolved in a minimum volume of phosphate buffer, usually 200 mL. In any case the smallest amount possible should be used. A small amount of the protease inhibitor (PMSF) is added to the buffer at $4^\circ$ C to inhibit proteases from degrading CCP. The dissolved pellets in the buffer with a protease inhibitor are dialyzed over night against 4 L of the buffer to remove the salt.

Overnight, insoluble material forms inside the dialysis bag. This material is not CCP. The solution inside the bag is centrifuged to get rid of the insoluble material. CCP is in the supernatant.

Next, the supernatant is subjected to two separate column chromatography steps to purify the CCP. CCP, under these conditions, is an anionic protein.

Anion Exchange Chromatography

The anion exchange column comprises a positively charged resin. Preferably, it is pre-equilibrated with the phosphate buffer. This is accomplished by pouring the buffer through the column several times. The protein will stick to the column after pre-equilibration. The dialyzed supernatant is poured onto and flows through the anion exchange column. CCP is a dark brown protein that sticks to the top of the column. A number of other proteins will come right through the column without sticking. After loading the column with the dialyzed supernatant, the column is washed with the buffer until the washings do not exhibit any absorbance at 280 nm as observed with a UV detector.

Any anion exchange resin can be used. There are quite a few commercial sources and variations of the anion exchange resins which can be used. For example, the Dowex 1-x series from Dow Chemical or the Cellex series from Biorad, or the DEAE-sephadex (or DEAE-sephacel or DEAE-sepharose) from Pharmacia. There are many more examples. In order to generalize, any resin with a functional group composed of DEAE (diethyl amino ethyl) or QAE (quaternary amino ethyl) or Q (quaternary amine), bound to any inert support such as cellulose, sephadex, sepharose or sephacryl, will be an appropriate resin. The preferred anion exchange column is available commercially under the name Q-sepharose from Pharmacia. It is a quaternary ammonium salt resin. It is preferred because it gives better yields of heme containing CCP than the DE-52 anion exchange resin.

CCP is still bound to the column with a few other proteins. The protein on the column is eluted with a 0.2 to 0.5 M linear salt gradient having a pH of at least 5. Any salt that is sufficiently soluble to form 0.5 M aqueous solution can be used, e.g. NaCl, $Na_2PO_4$, NaBr, $NH_4Cl$, etc. The pH level influences the salt concentration of the solution required to elute protein from the column. Higher pH requires higher salt concentrations. Different salt concentrations will replace different proteins on the anion exchange column with phosphate anions. The proteins coming off the column are monitored by monitoring absorbance at 280 nm, at the bottom of the column, versus fraction number, time or volume.

Experiments have established that CCP comes off the column at 0.4 M and above. This was determined by running the eluted fractions out on gel electrophoresis and looking for CCP by it's molecular weight or analyzing fractions with a CCP antibody or for enzyme activity. A quick way is to watch for the color of CCP in the eluates. The dark brown eluates will contain CCP.

The CCP fractions are desalted by a dialysis or ultra filtration or dilution at least 5 fold with water.

Affinity Chromatography Step

The desalted CCP containing fraction is put through a hydroxylapatite column, e.g. Biogel-HT commercial resin from Biorad. It has phosphate affinity. In the anion exchange column, phosphate was used as an anion. In this affinity column phosphate anion is used to elute the protein. In the anion exchange column we could have eluted the protein off with other anions, such as chloride. In the hydroxylapatite column only phosphate can be used because the column is a phosphate affinity column.

After loading the protein concentrate onto the Biogel-HT column which has been pre-equilibrated with phosphate buffer, the column is eluted with a 0.1 M to 0.4 M phosphate gradient. Pure CCP elutes off of the column at 0.25 M phosphate as a pure protein. This was established by gel electrophoresis. A single band on a gel electrophoresis at 34,000 molecular weight is obtained. This is confirmed by a CCP enzyme assay and also by UV spectra characteristic of CCP. The following example is presented to illustrate the present invention.

Example

One kg of dried baker's yeast was mixed to uniform consistency with 0.5 L of cold buffer A (10 mM phosphate, 1 mM EDTA, pH 6.0) + 0.2 mM PMSF. Then 0.5 L of ice cold ethyl acetate was added, again followed by mixing to uniform, paste-like consistency.

Autolysis was allowed to proceed for 6 hours at 4°C. To extract CCP from the lysate, 1.0 L of buffer A was added and the resulting suspension was stirred about 4 hours at 4°C. The suspension was centrifuged at 4°C. The supernatants were filtered through several layers of cheesecloth and pooled. A lipid material was floating on top of the supernatants. The pellets were resuspended in a minimal volume of buffer A and PMSF and this suspension was centrifuged as before and the supernatant pooled with the others.

The total volume of the cleared supernatant was measured (usually 1-2 L). Differential precipitation of the proteins in the pooled supernatant was achieved by slowly adding thereto solid ammonium sulfate up to 0.3 g/mL, which was about 50% saturation. When the ammonium sulfate was dissolved, the solution was allowed to stand in the cold for several hours or until two layers were formed. The top layer was ethyl acetate.

The top layer was removed and saved. The layers were centrifuged separately. Residue pellets were discarded. Solid ammonium sulfate (0.2 g/mL, now 80% saturated) was added to both the ethyl acetate fraction and the aqueous fraction. Both were allowed to stand in the cold for several hours. Two layers formed again. The two ethyl acetate layers and the two aqueous layers were combined separately.

These layers were centrifuged separately as before. The residue pellets were saved. The supernatants were discarded. The pellets were redissolved in a minimal volume of buffer A and PMSF. The resulting dark brown solution was placed in dialysis tubing (Spectrapor, 10-12 K cutoff), and dialyzed overnight against 4 L of buffer A containing PMSF. The dialyzed solution was centrifuged. The resulting pellet was discarded.

The supernatant was flowed through an anion exchange column (Q-sepharose from Pharmacia) which had been pre-equilibrated with buffer A (no PMSF). The column was washed extensively with buffer A (no CCP was removed). This was monitored through observing the protein UV absorption at 280 nm. Partially purified CCP was eluted from the column with a linear phosphate gradient [0.2 M phosphate (250 mL) to 0.5 M phosphate (250 mL), pH 6.0]. Fractions with CCP activity were dialyzed against buffer A (2 L), and applied to a hydroxylapatite column (Biogel HT from Biorad). The column was pre-equilibrated with buffer A.

Pure CCP was eluted from the column with a linear phosphate gradient [0.1 M phosphate (250 mL) to 0.4 M phosphate (250 mL), pH 6.0]. The purified CCP was stored in 50 mM phosphate buffer, pH 7.0 at -70°C. CCP purity was checked by SDS gel electrophoresis (1 band at 34,000 MW) and by uv-vis spectroscopy (ratio $A_{408}/A_{280}$ >1.20, and activity was determined using cytochrome c as a substrate. This procedure was completed in 3 days.

**Claims**

1. A method of purifying cytochrome c peroxidase from yeast characterized by the steps of:

 a) extracting a yeast lysate to obtain a crude protein extract comprising cytochrome c peroxidase;

 b) differentially precipitating the crude protein extract to obtain a precipitate comprising CCP;

 c) carrying out anion exchange chromatography on precipitate obtained in b) to obtain partially pure CCP; and

 d) carrying out phosphate affinity chromatography, with a hydroxyapatite column, on the partially pure CCP to obtain pure CCP.

2. A method of purifying cytochrome c peroxidase from yeast comprising the steps of:

 a) lysing the yeast;

 b) extracting the resulting lysate with a buffer having a pH of at least 5;

 c) treating the extract with increasing concentrations of ammonium sulfate solutions up to at least 80 % W/V to obtain differential salt precipitation of proteins in extract;

 d) dissolving protein containing precipitate obtained with at least 80% W/V ammonium sulfate solution in a buffer having i) a pH of at least 5 and ii) protease inhibitors dissolved therein;

 e) lowering the salt concentration in the solution of d);

 f) binding the proteins from the solution onto an anion exchange column;

 g) washing the column with a buffer having a pH of at least 5 until the washings are free of proteins;

 h) eluting the proteins from the column with a buffer having i) a pH of at least 5 and ii) a salt gradient concentration range of from 0.2 to at least 0.5 M;

i) lowering salt concentration in the eluates from h) having CCP activity;

j) binding protein from the eluates of i) onto a hydroxyapatite column; and

k) eluting pure CCP from the hydroxylapatite column with a linear phosphate gradient having a pH of 6 to 8 and a concentration ranging up to at least 0.4 M.

3. The method of claim 2 wherein the yeast is autolysed in step a) with a 1:1 mixture of i) a buffer having a pH of at least 5 and comprising a) a protease inhibitor and b) a metal chelator and ii) ethylacetate.

4. The method of claim 3 wherein up to 1Kg of dried yeast is autolysed in step a) wherein the buffer is a phosphate buffer having pH 6 and contains EDTA and phenylmethylsulfonyl chloride.

5. The method of claim 3 or claim 4 wherein up to 1 Kg of dried yeast is treated with 0.5 L of buffer and 0.5 L of ethyl acetate.

6. The method of any one of claims 2 to 5 wherein the protease inhibitor is phenylmethylsulfonyl chloride.

7. The method of any one of claims 2 to 6 wherein step e) is carried out by dialysis.

8. The method of any one of claims 2 to 7 wherein the anion exchange column used in step f) is pre-equilibrated with the buffer used as an eluant in step g).